# EUROPEAN PATENT APPLICATION

(11) **EP 1 640 007 A1**
(43) Date of publication of application: **29.03.2006**
(21) Application number: 04746827.7
(22) Date of filing: 25.06.2004
(51) Int. Cl.: A61K 31/522, A61P 25/08, C07D 473/06, C07D 473/12

(54) **ANTIEPILEPTIC AGENT**

(30) Priority: 27.06.2003 JP 2003185477
(71) Applicant: KYOWA HAKKO KOGYO CO., LTD., Chiyoda-ku, Tokyo 100-8185 (JP)
(72) Inventor: ICHIKAWA, Shunji Pharmaceutical Research Centre,, Nagaizumi-cho,Sunto-gun,Shizuoka 4118731 (JP); TAKASHIMA, Chiemi Pharmaceutical Research Center,, Nagaizumi-cho,Sunto-gun,Shizuoka 4118731 (JP); IMMA, Hironori c/o Head Office, Kyoka Hakko, Chiyoda-ku, Tokyo 100 8185, (JP); SHIMADA, Junichi Pharmaceutical Reasearch Center,, Nagaizumi-cho,Sunto-gun,Shizuoka 4118731 (JP)
(74) Representative: Vossius & Partner
(86) International application number: PCT/JP2004/009358
(87) International publication number: WO 2005/000313

(57) **Abstract**

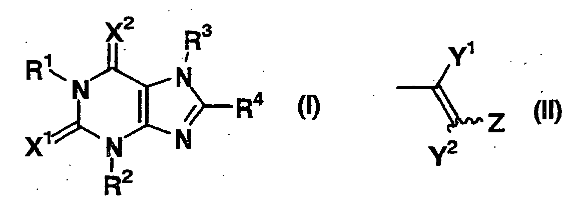

(wherein R¹, R² and R³ are the same or different and each represents a hydrogen atom, lower alkyl, lower alkenyl or lower alkynyl; R⁴ represents cycloalkyl,-(CH₂)ₙ-R⁵ or the above formula (II); and X¹ and X² are the same or different and each represents an oxygen atom or a sulfur atom)

An antiepil eptic agent comprising the xanthine derivative represented by the above formula (I) or a pharmaceutically acceptable salt thereof as an active ingredient is provided.

## Description

### Technical Field

The present invention relates to an antiepileptic agent comprising a xanthine derivative or a pharmaceutically acceptable salt thereof as an active ingredient.

### Background Art

According to the definition of WHO (the World Health Organization), "epilepsy" is "chronic brain dysfunction caused by various causes and its main characteristic is a repetitive seizure (epileptic seizure) caused by the result of excessive discharge of cerebral neurons, which is accompanied by various clinical symptoms and test observations". Symptom of the epileptic seizure is in various modes such as disturbance of consciousness, convulsion and automatism depending upon the initial site of sudden cerebral dysrhythmia and way of spreading thereof. In addition, in epilepsy, not only epileptic seizure but also, for example, periodic displeasure, episodic mental disorder, personality change, impairment of intelligence, etc. are often noted.

In treatment for suppressing the "epileptic seizure" as such, antiepileptic agents including that of a barbital type such as phenobarbital, that of benzodiazepine type such as clonazepam and valproic acid are used at present and it has been said that, in about 80% of patients suffering from epilepsy, the seizure can be completely suppressed by administration of drugs.

However, despite the fact that those antiepileptic agents used at present are very effective for suppression of the seizure, there are problems that they are accompanied by many side effects. For example, side effects such as central nervous system depression, cardiovascular collapse, aplastic anemia, visual hallucination, congestive heart failure, ataxia, personality change, psychosis, aggressive behavior, dizziness and sedation have been reported and there has been a demand for development of antiepileptic agents with reduced side effects.

Many of xanthine derivatives including the compound represented by the formula (I) which will be described later have been known to have, for example, anti-Parkinsonian action, central nerve exciting action and suppressive action on neurodegeneration (refer to Japanese Published Examined Patent Application No. 26,516/1972; Japanese Published Unexamined Patent Application No. 211,856/1994; Japanese Published Unexamined Patent Application No. 239, 862/1994; Japanese Published Unexamined Patent Application No. 016, 559/1994; WO 99/12546; etc.) . They have been also known to have antagonistic action to adenosine A₂ receptor, antidepressive action, anti-asthma action, suppressive action for bone resorption, hypoglycemic action, suppressive action for thrombocytosis, etc. (WO 92/06976; WO 94/01114; WO 95/23165; WO 99/35147; *Journal of Medicinal Chemistry,* 1991, volume 34, page 1431; *Journal of Medicinal Chemistry,* 1993, volume 36, page 1333).

### Disclosure of the Invention

An object of the present invention is to provide an excellent antiepileptic agent.

The present invention relates to the following (1) to (7).
(1) An antiepileptic agent comprising a xanthine derivative represented by the formula (I): 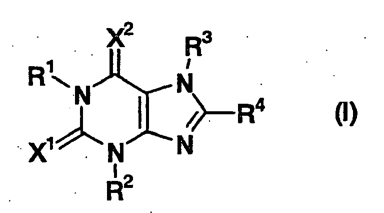 [wherein R¹, R² and R³are the same or different and each represents a hydrogen atom, lower alkyl, lower alkenyl or lower alkynyl;
   R⁴ represents cycloalkyl, - (CH₂)ₙ-R⁵ (wherein R⁵ represents substituted or unsubstituted aryl or substituted or a unsubstituted heterocyclic group and n represents an integer of 0 to 4) or the formula (II): 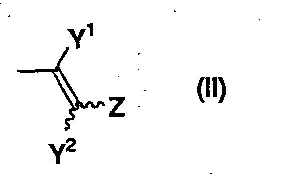 (wherein Y¹ and Y² are the same or different and each represents a hydrogen atom, halogen or lower alkyl and Z represents substituted or unsubstituted aryl or substituted or unsubstituted heterocyclic group); and
   X¹ and X² are the same or different and each represents an oxygen atom or a sulfur atom] or a pharmaceutically acceptable salt thereof as an active ingredient.
(2) The antiepileptic agent according to the above (1),
   wherein X¹ and X² are oxygen atoms.
(3) The antiepileptic agent according to the above (1) or (2), wherein R⁴ is the formula (II): 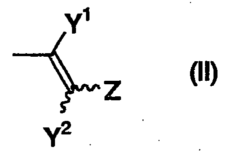 (wherein Y¹, Y² and Z have the same meanings as defined above, respectively).
(4) The antiepileptic agent according to the above (3),
   wherein Y¹ and Y² are hydrogen atoms.
(5) The antiepileptic agent according to the above (3) or (4), wherein Z is substituted or unsubstituted aryl or the formula (III): 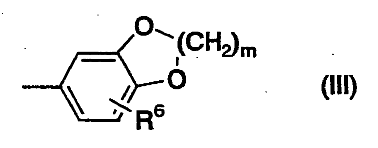 (wherein R⁶ represents a hydrogen atom, hydroxy, lower alkyl, lower alkoxy, halogen, nitro or amino; and m represents an integer of 1 to 3).
(6) A method for treating epilepsy, which comprises administering an effective amount of the xanthine derivative represented by the formula (I): 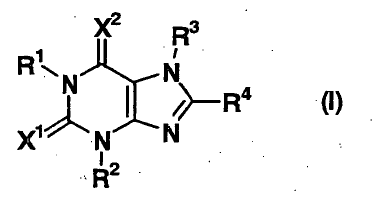 (wherein R¹, R², R³, R⁴ X¹ and X² have the same meaning as defined above, respectively) or a pharmaceutically acceptable salt thereof.
(7) Use of a xanthine derivative represented by the formula (I): 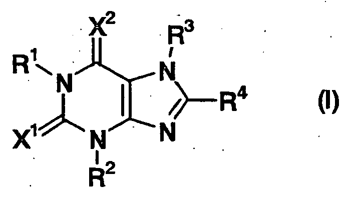 (wherein R¹, R², R³, R⁴, X¹ and X² have the same meaning as defined above, respectively) or a pharmaceutically acceptable salt thereof for the manufacture of an antiepileptic agent.

Hereinafter, the compound represented by the formula (I) is referred to as Compound (I).

In the definition for each group of the formula (I):
Examples of a lower alkyl moiety in the lower alkyl and the lower alkoxy include linear or branched alkyl having 1 to 6 carbon(s), and specific examples thereof include methyl, ethyl, propyl, isopropyl, butyl, isobutyl, sec-butyl, tert-butyl, pentyl, neopentyl, hexyl and the like.

Examples of the lower alkenyl include linear or branched alkenyl having 2 to 6 carbons, and specific examples thereof include vinyl, allyl, methacryl, crotyl, 3-butenyl, 2-pentenyl, 4-pentenyl, 2-hexenyl, 5-hexenyl and the like.

Examples of the lower alkynyl include linear or branched alkynyl having 2 to 6 carbons, and specific examples thereof include ethynyl, propargyl, 2-butynyl, 3-butynyl, 2-pentynyl, 4-pentynyl, 2-hexynyl, 5-hexynyl, 4-metyl-2-pentynyl and the like.

Examples of the cycloalkyl include a cycloalkyl having 3 to 8 carbons, and specific examples thereof include cyclopropyl, cyclobutyl, cyclopentyl, cyclohexyl, cycloheptyl, cyclooctyl and the like.

The halogen means each of fluorine, chlorine, bromine and iodine atoms.

Examples of the aryl include an aryl having 6 to 14 carbons, and specific examples thereof include phenyl, naphthyl, anthryl and the like.

Examples of the heterocyclic group include five- or six-membered monocyclic heterocyclic groups containing at least one atom selected from a nitrogen atom, an oxygen atom and a sulfur atom; bicyclic or tricyclic condenced-ring heterocyclic groups at least one atom selected from a nitrogen atom, an oxygen atom and a sulfur atom in which 3- to 8-membered rings are condensed and the like. Specific examples thereof include furyl, thienyl, pyrrolyl, pyranyl, thiopyranyl, pyridyl, pyrimidinyl, triazinyl, purinyl, pyrazinyl, pyridazinyl, benzoimidazolyl, 2-oxobenzoimidazolyl, benzotriazolyl, benzofuryl, benzothienyl, benzoxazolyl, benzothiazolyl, 1,3-benzodioxolyl, 1,4-benzodioxanyl, 3,4-dihydro-2H-1,5-benzodioxepinyl, indazolyl, indolyl, isoindolyl, quinolyl, isoquinolyl, phthalazinyl, naphthylidinyl, quinoxalinyl, pyrazolyl, quinazolinyl, cinnolinyl, triazolyl, tetrazolyl, imidazolyl, oxazolyl, isoxazolyl, thiazolyl, isothiazolyl, dihydroisoquinolyl, tetrahydroquinolyl, benzodihydropyranyl and the like.

The substituted aryl and the substituted heterocyclic group have 1 to 3 substituents which are the same or different, such as lower alkyl, lower alkenyl, lower alkynyl, hydroxy, substituted or unsubstituted lower alkoxy, halogen, nitro, amino, lower alkylamino, di-lower alkylamino, trifluoromethyl, trifluoromethoxy, aralkyl, aralkyloxy, aryl, aryloxy, lower alkanoyl, lower alkanoyloxy, aroyl, aroyloxy, arylalkanoyloxy, carboxy, lower alkoxycarbonyl, lower alkylcarbamoyl, di-lower alkylcarbamoyl, sulfo, lower alkoxysulfonyl, lower alkylsulfamoyl and di-lower alkylsulfamoyl.

The lower alkyl moiety of the above-described lower alkyl, lower alkoxy, lower alkylamino, di-lower alkylamino, lower alkanoyl, lower alkanoyloxy, lower alkoxycarbonyl, lower alkylcarbamoyl, di-lower alkylcarbamoyl, lower alkoxysulfonyl, lower alkylsulfamoyl and di-lower alkylsulfamoyl has the same meaning as the above-described lower alkyl. The halogen, the lower alkenyl and the lower alkynyl have the same meanings as described above, respectively. Two lower alkyl moieties of the di-lower alkylamino, the di-lower alkylcarbamoyl and the di-lower alkylsulfamoyl may be the same or different. An aryl moiety of the aryl and the aryloxy is the same as the above-described aryl, and examples of the aralkyl moiety of the aralkyl and the aralkyloxy include benzyl, phenethyl and the like. Examples of an aroyl moiety in the aroyl and the aroyloxy include benzoyl, naphthoyl and the like. Examples of an arylalkyl moiety of the arylalkanoyloxy are benzyl and phenethyl. Examples of the substituent (s) in the substituted lower alkoxy include hydroxy, lower alkoxy, halogen, amino, azido, carboxy, lower alkoxycarbonyl and the like. Herein, a lower alkyl moiety in the lower alkoxy and the lower alkoxycarbonyl has the same meaning as the above-described lower alkyl, and the halogen has the same meaning as described above.

Examples of the pharmaceutically acceptable salt of Compound (I) are pharmaceutically acceptable acid addition salt, metal salt, ammonium salt, organic amine addition salt, amino acid addition salt and the like.

Examples of the pharmaceutically acceptable acid addition salts of Compound (I) include an inorganic acid salt such as hydrochloride, sulfate and phosphate; and an organic acid salt such as acetate, maleate, fumarate, tartrate, citrate and methanesulfonate. Examples of the pharmaceutically acceptable metal salts include an alkali metal salt such as sodium salt and potassium salt; alkaline earth metal salt such as magnesium salt and calcium salt; aluminum salt; zinc salt and the like. Examples of the pharmaceutically acceptable ammonium salts include ammonium and tetramethylammonium. Examples of the pharmaceutically acceptable organic amine addition salt include an addition salt of morpholine or piperidine. Examples of the pharmaceutically acceptable amino acid addition salts include an addition salt of lysine, glycine or phenylalanine.

Compound (I) is able to be produced by a process disclosed in Japanese Published Examined Patent Application No. 26, 516/1972, *Journal of Medicinal Chemistry,* 1991, volume 34, page 1431, *Journal of Medicinal Chemistry,* 1993, volume 36, page 1333, WO 92/06976, Japanese Published Unexamined Patent Application No. 211, 856/1994, Japanese Published Unexamined Patent Application No. 239,862/1994, WO 95/23165, Japanese Published Unexamined Patent Application No. 16559/1994, WO 94/01114, WO 99/12546, WO 99/35147 and the like, or by a process similar thereto.

The aimed compound in each production process can be isolated and purified by a purifying method which has been commonly used in synthetic organic chemistry such as filtration, extraction, washing, drying, concentration, recrystallization and various chromatographic means.

When it is desired to obtain a salt of Compound (I), in the case where Compound (I) is produced in the form of the salt, it can be purified as it is, but where it is produced in its free form, it can be converted into a salt, after being dissolved or suspended in an appropriate solvent followed by adding an appropriate acid or base.

Furthermore, Compound (I) and pharmaceutically acceptable salts thereof may exist in the form of adducts with water or various solvents, and these adducts are also used for the antiepileptic agent of the present invention.

For some of Compounds (I), there may exist optical isomers and the like, and all possible isomers including them and mixtures thereof may be used for the antiepileptic agent of the present invention.

Specific examples of Compound (I) are shown in Table 1.

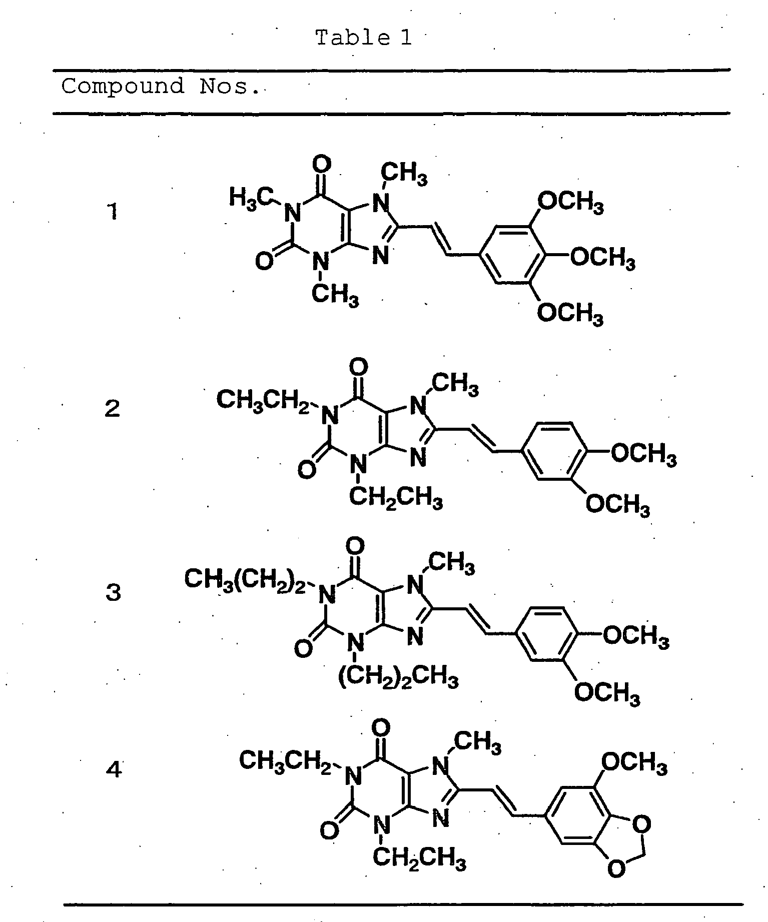

### Compound 1: (E) -8- (3, 4, 5-Trimethoxystyryl) caffeine (Japanese Published Examined Patent Application No. 26516/1972) IR (KBr) vmax(cm⁻¹) : 1702, 1667, 1508, 1432

NMR (DMSO-d₆, 27OMHz) δ(ppm) : 7.61(1H, d, J = 16.0Hz), 7.25 (1H, d, J = 16.0Hz), 7.09 (2H, s), 4.03 (3H, s), 3.85 (6H, s), 3.71 (3H, s), 3.45 (3H, s), 3.21 (3H, s)
MS (EI) 386(M⁺)

### Compound 2: (E)-8-(3,4-Dimethoxystyryl)-1,3-diethyl-7-methylxanthine (Japanese Published Unexamined Patent Application No. 211,856/1994)

Melting point: 190.4-191.3°C

Elementary analysis as C₂₀H₂₄N₄O₄

Calculated (%): C 62.48, H 6.29, N 14.57

Found (%): C 62.52, H 6.53, N 14.56

IR (KBr) vmax (cm⁻¹): 1697, 1655, 1518

NMR (CDCl₃, 270MHz) δ(ppm): 7.74 (1H, d, J = 15.5Hz), 7.18 (1H, dd, J = 8.3, 1.9Hz), 7.08 (1H, d, J =1.9Hz), 6. 89 (1H, d, J = 8.3Hz), 6.77 (1H, d, J = 15.5Hz), 4.21 (2H, q, J = 6.9Hz), 4.09 (2H, q, J = 6.9Hz), 4.06 (3H, s), 3.96 (3H, s), 3.93 (3H, s), 1.39 (3H, t, J = 6.9Hz), 1.27 (3H, t, J = 6.9Hz).

### Compound 3: (E)-8-(3,4-Dimethoxystyryl)-7-methyl-1,3-dipropylxanthine (WO 92/0.6976)

Melting point: 164.8-166.2°C (recrystallized from 2-propanol/water)

Elementary analysis for C₂₂H₂₈N₄O₄

Calculated (%): C 64.06, H 6.84, N 13.58

Found (%): C 64.06, H 6.82, N 13.80

IR (KBr) vmax(cm⁻¹): 1692, 1657

NMR (DMSO-d₆, 270MHz) δ(ppm): 7.60 (1H, d, J = 15.8Hz), 7.40 (1H, d, J = 2.0Hz), 7.28 (1H, dd, J = 2.0, 8.4Hz), 7.18 (1H, d, J = 15. 8Hz), 6.99 (1H, d, J = 8.4Hz), 4.02 (3H, s), 3.99 (2H, t), 3.90-3.80 (2H, m), 3.85 (3H, s), 3.80 (3H, s), 1.85-1.50 (4H, m), 1.00-0.85 (6H, m)

### Compound 4: (E)-1,3-Diethyl-8-(3-methoxy-4,5-methylenedioxystyryl)-7-methylxanthine (Japanese Published Unexamined Patent Application No. 211,856/1994)

Melting point: 201.5-202.3°C

Elementary analysis for C₂₀H₂₂N₄Oₛ

Calcd (%): C 60.29, H 5.57, N 14.06

Found (%): C 60.18, H 5.72, N 13.98

IR (KBr) vmax(cm⁻¹): 1694, 1650, 1543, 1512, 1433

NMR (DMSO-d₆, 270MHz) δ(ppm): 7.58 (1H, d, J = 15.8Hz), 7.23 (1H, d, J = 15.8Hz), 7.20 (1H, d, J = 1.0Hz), 7.09 (1H, d, J = 1.0Hz), 6.05 (2H, s), 4.09-4.02 (2H, m), 4.02 (3H, s), 3.94-3.89 (2H, m), 3.89 (3H, s), 1.25 (3H, t, J = 7.2Hz), 1.13 (3H, t, J = 6.9Hz)

Next, the pharmacological action of Compound (I) will be illustrated by way of Test Examples.

### Test Example 1: Antiepileptic action

Convulsion induced by administration of reserpine and electric stimulation has been known as an effective model for epilepsy [*European Journal of Pharmacology,* 1994, volume 23, page 260].

Male mice (body weight: 15 to 17g; Nippon SLC, Hamamatsu) of ddY strain of four weeks age were subjected to a preliminary breeding and 65 mice where body weight became 22.4 to 26.0 g were used for the experiment. Fifteen mice among the above were used as a non-treated group. Reserpine (Apoplon Injection 1 mg; Daiichi Seiyaku) diluted with distilled water for injection (Otsuka Seiyaku, Naruto) was intraperitoneally administered (treatment with reserpine) at the dose of 5 mg/kg to each of the remaining 50 mice and the experiment was carried out after 18 to 24 hours from the administration.

Among the mice subjected to the treatment with reserpine, two groups each comprising ten mice were randomly selected and used as a group administered with a solvent and a group administered with the test compound.

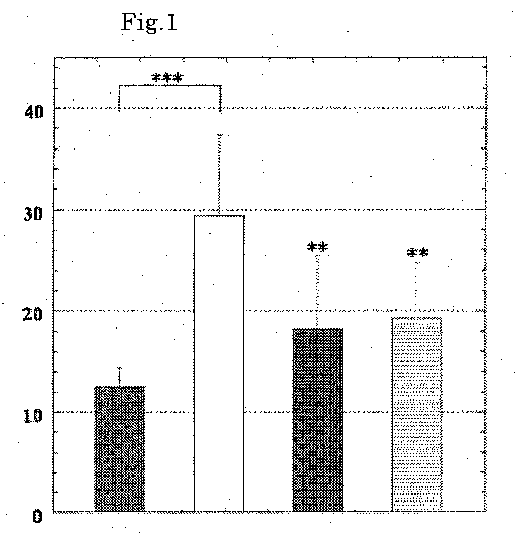

The test compound was used as a suspension of a 0.5% MC solution [distilled water for injection containing 0.5% methyl cellulose 400 cP (Wako Pure Chemicals)] and Compound (I) was orally administered at the dose of 30 mg/kg (10 mL/kg in terms of volume) to the mice treated with reserpine (a group administered with the test compound). On the other hand, only 0.5% MC solution was orally administered at the dose of 10 ml/kg to the mice treated with reserpine as a group administered with solvent. After one hour, electric stimulation of 1,000 V, 50 mA and 0.2 second was applied to the mice using an induction device for electric convulsion (type: USA-201; Unique Medical) to induce tonic extended convulsion and the duration in the group administered with the test compound was compared with that of the non-treated group and the group administered with solvent. The result is shown in Fig. 1.

As shown in Fig. 1, duration of convulsion was significantly extended (p < 0.001; Student's -test) in the group treated with reserpine and administered with solvent as compared with a non-treated group. On the other hand, in a group administered with the test compound to which the compound 1 or 3 was administered, a significant shortening action was noted in terms of duration of convulsion as compared with the group which was treated with reserpine and administered with solvent (p < 0.01; Aspine-Welch test).

Since Compound (I) or a pharmaceutically acceptable salt thereof has an antiepileptic action, they are suggested to be effective for prevention and/or treatment of epilepsy, epilepsy-based diseases and the like.

### Test Example 2: Acute toxicity test

A test compound was orally administered to male mice (body weight: 20 ± 1 g) of dd-strain which comprises three mice per group. State of death after 7 days from the administration was observed and the minimum lethal dose (MLD) was determined.

As a result, the MLD of the compound 1 was >1,000 mg/kg and its toxicity is believed to be very low.

Examples of epilepsy which can be prevented and/or treated by administrating the antiepileptic agent of the present invention are essential epilepsy without a known cause which has been generally called epileptic seizure, symptomatic epilepsy caused by intracerebral lesion (such as Jackson epilepsy, West syndrome, Lennox-Gastaut syndrome and the like) and febrile convulsion and the like. Examples of the epileptic seizure are (1) those which are classified as partial (focal or topic) seizure such as (i) simple partial seizure, (ii) complex partial seizure and (iii) general seizure which secondarily progressed from partial seizure, (2) those which are classified as convulsive and non-convulsive general seizure such as (i) absence seizure, (ii) myoclonus seizure, (iii) clonic seizure, (iv) tonic seizure, (v) tonic clonic seizure and (vi) cataplexy and (3) accumulated epileptic seizure.

The antiepileptic agent of the present invention is suitably used particularly for the seizure accompanied by convulsion and, to be more specific, it is able to be suitably used for prevention and/or treatment of simple partial seizure accompanied by convulsion, tonic seizure, clonic seizure, tonic clonic seizure or myoclonus seizure and in the case where such seizures compositely take place such as grand mal, Jackson epilepsy, West syndrome, Lennox-Gastaut syndrome and febrile convulsion.

Compound (I) or a pharmaceutically acceptable salt thereof may be used either as it is or in various pharmaceutical dosage forms. The pharmaceutical composition of the present invention may be manufactured by a uniform mixing of Compound (I) or a pharmaceutically acceptable salt thereof as an active ingredient in an effective dose with a pharmaceutically acceptable carrier. It is preferred that such a pharmaceutical composition is in a unit dosage form suitable for the administration such as rectal administration or oral or parenteral (including subcutaneous, intravenous and intramuscular) administration.

In preparing a composition in an orally administering form, any useful pharmaceutically acceptable carrier may be used. In the case of oral liquid preparation such as suspension and syrup, it may be manufactured using water, saccharide such as sucrose, sorbitol and fructose, glycol such as polyethylene glycol and propylene glycol, oil such as sesame oil, olive oil and soybean oil, antiseptic agent such as p-hydroxybenzoate, flavor such as strawberry flavor and peppermint, etc. In the case of diluted powder, pill, capsule and tablet, it may be prepared using excipient such as lactose, glucose, sucrose and mannitol, disintegrating agent such as starch and sodium alginate, lubricant such as magnesium stearate and talc, binder such as polyvinyl alcohol, hydroxypropyl cellulose and gelatin, surfactant such as fatty acid ester, plasticizer such as glycerol, etc. Tablets and capsules are the most useful unit agents being administered per os because their administration is easy. In the manufacture of tablets and capsules, a solid pharmaceutical carrier is used.

Injection preparation can be prepared using a carrier comprising distilled water, salt solution, glucose solution or a mixture of brine and glucose solution. In that case, it is prepared as solution, suspension or dispersion using an appropriate adjuvant according to the conventional method.

Compound (I) or a pharmaceutically acceptable salt thereof can be administered orally in the above-described pharmaceutical dosage form or parenterally as injection, etc. Although effective dose and administering frequency thereof vary depending upon administration form, age and body weight of a patient, symptom, etc., it is appropriate to administer 1 to 100 mg/60 kg/day or, preferably, 1 to 20 mg/60 kg/day once daily or several times a day.

### Brief Description of the Drawings

Fig. 1 shows the action of the compound 1 and the compound 3 on extension of duration of tonic extensive convulsion by administration of reserpine and electric stimulation. An ordinate shows duration (in seconds) of tonic extensive convulsion and bar graphs show the cases of non-treated group, group to which solvent is administered, group to which the compound 1 is administered and group to which the compound 3 is administered from left to right. Meanings of the symbols in the graph are as follows.
   ***: statistically significant difference where p < 0.001 (Student's -test)
   **: statistically significant difference where p < 0.01 (Aspine-Welch test)

### Best Mode for Carrying Out the Invention

Embodiments of the present invention are illustrated in detail below referring to examples.

### Example 1: Tablets

Tablets comprising the following composition are prepared by a conventional method.

The compound 1 (40 g), 286.8 g of lactose and 60 g of potato starch are mixed and 120 g of a 10% aqueous solution of hydroxypropyl cellulose is added thereto. The mixture is kneaded by a conventional method, granulated, dried and subjected to particle size selection to give granules for making into tablets. Magnesium stearate (1.2 g) is added thereto and mixed therewith and subjected to tabletting using a tabletting machine (RT-15 manufactured by Kikushisha) having punches with 8 mm diameter to give tablets (each tablet contained 20 mg of the active ingredient).

**Formulation**

| | |
|---|---|
| Compound 1 | 20 mg |
| Lactose | 143.3 mg |
| Potato starch | 30 mg |
| Hydroxypropyl cellulose | 6 mg |
| Magnesium stearate | 0.6 mg |
| | 200 mg |

### Example 2: Capsule preparations

Capsule preparations comprising the following composition are prepared by a conventional method.

The compound 2 (200 g), 995 g of Avicel and 5 g of magnesium stearate are mixed by a conventional method. The mixture is filled in hard capsules No. 4 (capacity of one capsule is 120 mg) using a capsule filling machine (type LZ-64; manufactured by Zanasi) to prepare capsule preparations (each capsule contained 20 mg of the active ingredient).

**Formulation**

| | |
|---|---|
| Compound 2 | 20 mg |
| Avicel | 99.5 mg |
| Magnesium stearate | 0.5 mg |
| | 120 mg |

### Example 3: Injection preparations

Injection preparations comprising the following composition are prepared by a conventional method.

The compound 3 (1 g) is dissolved in 100 g of pure soybean oil and 12 g of pure yolk lecithin and 25 g of glycerol for injection are added thereto. The mixture is made 1,000 mL using distilled water for inj ection by a conventional method followed by kneading and emulsifying. The resulting dispersion is subjected to an aseptic filtration using a membrane filter of a disposable type of 0.2 µm and each 2 mL thereof is aseptically filled in a glass vial to prepare injection preparations (each vial contained 2 mg of the active ingredient).

**Formulation**

| | |
|---|---|
| Compound 3 | 2 mg |
| Pure soybean oil | 200 mg |
| Pure yolk lecithin | 24 mg |
| Glycerol for injection | 50 mg |
| Distilled water for injection | 1.72 mL |
| | 2.00 mL |

### Example 4: Suppositories for anus

A preparation for rectal administration comprising the following composition is prepared by a conventional method.

Witepsol ™ H15 (manufactured by Dynamite Nobel) (678.8 g) and 290.9 g of Witepsol™ E75 (manufactured by Dynamite Nobel) are melted at 40 to 50□. The compound 4 (2.5 g), 13.6 g of potassium primary phosphate and 14.2 g of sodium secondary phosphate are uniformly mixed with the above and dispersed therein. After that, the mixed/dispersed product is filled in suppository molds made of plastics followed by gradual cooling to prepare suppositories for anus (each preparation contained 2.5 mg of the active ingredient).

**Formulation**

| | |
|---|---|
| Compound 4 | 2.5 mg |
| Witepsol™ H15 | 678.8 mg |
| Witepsol™ E75 | 290.9 mg |
| Potassium primary phosphate | 13.6 mg |
| Sodium secondary phosphate | 14.2 mg |
| | 1,000 mg |

### Industrial Applicability

In accordance with present invention, an antiepileptic agent comprising a xanthine derivative or a pharmaceutically acceptable salt thereof as an active ingredient is provided.

## Claims

1. An antiepileptic agent comprising a xanthine derivative represented by the formula (I) : 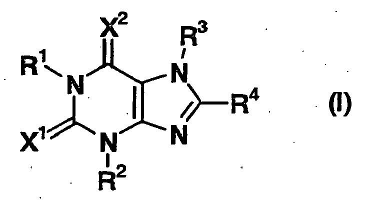 [wherein R¹, R² and R³ are the same or different and each represents a hydrogen atom, lower alkyl, lower alkenyl or lower alkynyl;
R⁴ represents cycloalkyl, -(CH₂)ₙ-R⁵(wherein R⁵ represents substituted or unsubstituted aryl or substituted or unsubstituted heterocyclic group and n represents an integer of 0 to 4) or the formula (II): 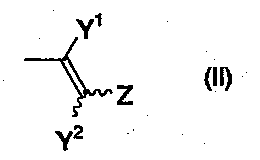 (wherein Y¹ and Y² are the same or different and each represents a hydrogen atom, halogen or lower alkyl and Z represents substituted or unsubstituted aryl or substituted or unsubstituted heterocyclic group); and
X¹ and X² are the same or different and each represents an oxygen atom or a sulfur atom] or a pharmaceutically acceptable salt thereof as an active ingredient.

2. The antiepileptic agent according to claim 1, wherein X¹ and X² are oxygen atoms.

3. The antiepileptic agent according to claim 1 or 2,
wherein R⁴ is the formula (II): 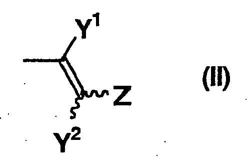 (wherein Y¹, Y² and Z have the same meanings as defined above, respectively) .

4. The antiepileptic agent according to claim 3, wherein Y¹ and Y² are hydrogen atoms.

5. The antiepileptic agent according to claim 3 or 4, wherein Z is substituted or unsubstituted aryl or the formula (III) : 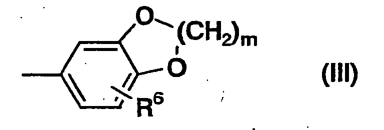 (wherein R⁶ represents a hydrogen atom, hydroxy, lower alkyl, lower alkoxy, halogen, nitro or amino; and m represents an integer of 1 to 3).

6. A method for treating epilepsy, which comprises administering an effective amount of a xanthine derivative represented by the formula (I): 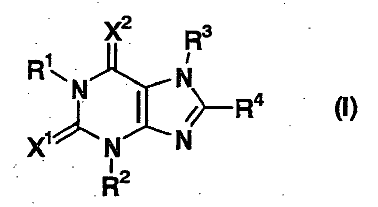 [wherein R¹, R² and R³ are the same or different and each represents a hydrogen atom, lower alkyl, lower alkenyl or lower alkynyl;
R⁴ represents cycloalkyl, - (CH₂) ₙ-R⁵ (wherein R⁵ is substituted or unsubstituted aryl or substituted or unsubstituted heterocyclic group and n represents an integer of 0 to 4) or the formula (II): 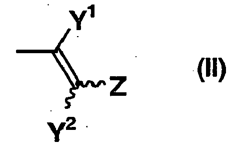 (wherein Y¹ and Y² are the same or different and each represents a hydrogen atom, halogen or lower alkyl and Z represents substituted or unsubstituted aryl or substituted or unsubstituted heterocyclic group); and
X¹ and X² are the same or different and each represents an oxygen atom or a sulfur atom] or a pharmaceutically acceptable salt thereof.

7. Use of a xanthine derivative represented by the formula (I): 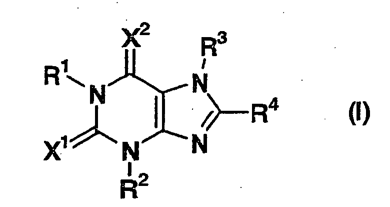 [wherein R¹, R² and R³ are the same or different and each represents a hydrogen atom, lower alkyl, lower alkenyl or lower alkynyl;
R⁴ represents cycloalkyl, -(CH₂)ₙ-R⁵ (wherein R⁵ represents substituted or unsubstituted aryl or substituted or unsubstituted heterocyclic group and n represents an integer of 0 to 4) or the formula (II): 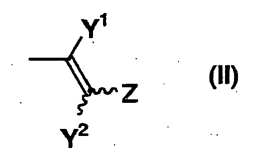 (wherein Y¹ and Y² are the same or different and each represents hydrogen atom, halogen or lower alkyl and Z represents substituted or unsubstituted aryl or substituted or unsubstituted heterocyclic group); and
X¹ and X² are the same or different and each represents an oxygen atom or a sulfur atom] or a pharmaceutically acceptable salt thereof, for the manufacture of an antiepileptic agent.
